# EUROPEAN PATENT APPLICATION

(11) **EP 4 378 374 A1**
(43) Date of publication of application: **05.06.2024**
(21) Application number: 22210941.5
(22) Date of filing: 01.12.2022
(51) Int. Cl.: A61B 5/00

(54) **A SYSTEM FOR DETECTING BRUXISM OR OTHER JAW DISORDERS**

(71) Applicant: Koninklijke Philips N.V., 5656 AG Eindhoven (NL)
(72) Inventor: JOHNSON, Mark Thomas, Eindhoven (NL); GERHARDT, Lutz Christian, Eindhoven (NL); SPELT, Hanne Adriana Alijda, Eindhoven (NL); KOOIJMAN, Gerben, Eindhoven (NL); DE RUYTER, Boris Emmanuel Rachmund, Eindhoven (NL)
(74) Representative: Philips Intellectual Property & Standards

(57) **Abstract**

A system is provided for diagnosing, or predicting a risk of developing, bruxism or other jaw disorders, comprising: a receiver for receiving audio data and video data of a subject; a processor configured to: identify one of the audio data and video data indicative of the subject having, or a risk of the subject developing, bruxism or other jaw disorders, and in response thereto, analyze the other of the audio data and video data to diagnose the subject having, or predict the risk of the subject developing, bruxism or other jaw disorders. Further, a computer program which is adapted, when said program is run on a processor, to implement a method for diagnosing or, predicting a risk of developing, bruxism or other jaw disorders is also provided.

## Description

### FIELD OF THE INVENTION

The invention relates to a system for detecting or diagnosing whether a subject has bruxism or other jaw disorders, in particular in a non-obtrusive and user-friendly manner. The invention also relates to a system for predicting whether a subject has a tendency or risk for developing bruxism or other jaw disorders, in particular in a non-obtrusive and user-friendly manner.

### BACKGROUND OF THE INVENTION

The jawbones (the upper, maxilla and the lower, mandible) are integral parts of the oral cavity. These bones are important for normal functioning of the oral cavity, and have been also associated with pathological conditions in oral cavity. Bruxism is a condition in which a subject grinds, gnashes or clenches the teeth. Temporomandibular joint disorder is another condition linked with the jawbones.

Bruxism can be categorized as sleep bruxism and awake bruxism. Sleep bruxism is characterized by rhythmic or non-rhythmic muscle activities of the biting apparatus during sleep. Awake bruxism is characterized by repetitive or sustained tooth contact and/or by bracing or thrusting/sliding of the lower jawbone. The prevalence of bruxism varies according to the age group. In the adult population, the prevalence of awake bruxism is estimated to be around 22%-30%, and sleep bruxism around 1%-15% of the population. In children and adolescents the prevalence of bruxism is reported to be around 3%-49%.

Bruxism is associated with a plethora of short and long-term adverse health effects. Short-term effects of bruxism include headache, limitation of mouth opening, facial myalgia (aching jaw & facial muscles), earache, and tightness or stiffness of the shoulders. Bruxism also impacts sleep in the form of frequent disruptions. It is also known that the grinding or tapping noise of the teeth due to bruxism can lead to sleep disruption of a bed partner. The oral manifestations of bruxism include excess tooth mobility and inflamed and receding gums. The long-term effects of bruxism include Temporomandibular Joint Disorders (hereinafter referred to as TMJD, discussed further below), tooth wear and fractures. TMJD is a group of more than 30 conditions that cause pain and dysfunction in the jaw joint and muscles that control jaw movement. TMJ disorders can also cause a clicking sound or grating sensation when a subject opens their mouth or chews.

If diagnosed and treated early, many bruxism-related problems can be avoided. It has for example been observed that awake bruxism can be reduced by using proper reminder techniques.

This invention relates in one particular example to awake bruxism, where a subject clenches or grinds their teeth without necessarily being aware of doing this. It would be desirable to be able to diagnose such bruxism or to predict whether the subject will develop such bruxism, for example to enable a timely alert to be provided to a user to stop teeth grinding or clenching. More generally, it would be desirable to have an effective way to detect jaw disorders.

### SUMMARY OF THE INVENTION

The invention is defined by the claims.

According to examples in accordance with an aspect of the invention, a system for diagnosing, or predicting a risk of developing, bruxism or other jaw disorders, comprising: a receiver for receiving audio data and video data of a subject; a processor configured to: identify or analyze one of the audio data and video data indicative of the subject having, or a risk of the subject developing, bruxism or other jaw disorders, and in response thereto, analyze the other of the audio data and video data to diagnose the subject having, or predict the risk of the subject developing, bruxism or other jaw disorders.

According to examples in accordance with an aspect of the invention, a system for diagnosing bruxism or other jaw disorders is presented, comprising: a receiver for receiving audio data and video data of a subject; a processor configured to: identify or analyze one of the audio data and video data indicative of the subject having bruxism or other jaw disorders, and in response thereto, analyze the other of the audio data and video data to diagnose the subject having bruxism or other jaw disorders.

According to examples in accordance with an aspect of the invention, a system for predicting a risk of developing bruxism or other jaw disorders is presented, comprising: a receiver for receiving audio data and video data of a subject; a processor configured to: identify or analyze one of the audio data and video data indicative of the subject developing bruxism or other jaw disorders, and in response thereto, analyze the other of the audio data and video data to predict the risk of the subject developing bruxism or other jaw disorders.

This system aims to diagnose, or predict a risk or tendency of developing, bruxism or other jaw disorders, by analysis of audio and video data of a subject. As a result, it can be performed remotely, enabling the subject to be at home while the analysis takes place. It may for example be implemented as part of a video conference system.

The use of audio and video analysis increases the specificity of the bruxism or other jaw disorder diagnosis or the specificity of the risk of developing bruxism or other jaw disorder, while enabling an efficient use of resources, e.g. only using processing power when needed.

For example, the data volume to be processed can be greatly reduced because at least some measurements are only performed when the greatest effect can be expected, because a suitable analysis of one of the data streams is performed depending on what has been detected in the other data stream (e.g., during yawning and sneezing, a maximum mouth opening may occur - abruptly in the case of sneezing - which can be detected in a video stream, and then popping sounds of the jaw joint can be detected in the audio stream).

A more specific diagnosis or prediction can be made by combining analysis of the video and audio data. The sound analysis for example may be used to detect sneezing or it may be used to perform general sound and speech analysis.

The processor is for example configured to: identify or analyze the video data indicative of the subject having, or the risk of the subject developing, bruxism or other jaw disorders by identifying a prolonged period of closed jaws whilst the lips of the user are observed to move; and in response, analyze the audio data to determine if the subject is speaking.

By observing the video alone, it would not be clear that the closed jaw is related to a clenching of the jaws, or simply a result of somebody not talking or indeed talking through a closed, but not clenched, jaw. Thus, the added specific audio analysis gives further resolution to the diagnosis process or prediction of risk of developing bruxism or other jaw disorders.

The processor may then (if speech is detected) be configured to analyze the spectra of the audio data to detect speech features which are characteristic of bruxism.

The processor may in another example be configured to analyze or identify the video data to detect yawning as the indication of the subject having, or the risk of the subject developing, bruxism or other jaw disorders; and in response, analyze the audio data to detect popping or clicking sounds.

These sounds are indicative of a TMJD which itself may result from bruxism, and hence is an example of another jaw disorder.

The processor may be configured to: identify or analyze the audio data indicative of the subject having, or the risk of the subject developing, bruxism or other jaw disorders by detecting speech sounds characteristic of speech with a fixed jaw position; and in response, analyze the video data to determine if the subject is speaking with a closed mouth.

A closed mouth is for example identified when the upper and lower teeth are in contact with each other. A clenched mouth will additionally have jaw or cheek muscle contraction as discussed below.

If a subject is speaking with a closed mouth, the chance of the user having daytime bruxism is higher. In particular, during episodes of daytime bruxism, the subject will talk whilst clenching the jaws together. The lack of jaw opening will be identified in the video data, to confirm the audio analysis.

The processor may be configured to analyze the video data to diagnose the subject having, or predict the risk of the subject developing, bruxism or other jaw disorders by identifying recurring sideways grinding motions of a jaw.

The processor may be configured to analyze the video data to diagnose the subject having, or predict the risk of the subject developing, bruxism or other jaw disorders by monitoring facial skin and/or muscle motion at the position of the joint of the jaw and/or movement of the ears to detect jaw clenching.

There may be pulsating cheek muscle motion (even sometimes pulsating skin motion on the temples) due to clenching. Fast Fourier Transform analysis may for example be performed on the image to extract higher frequency content (>2 Hz) from a video sequence.

The processor may be configured to analyze the audio data to diagnose the subject having, or predict the risk of the subject developing, bruxism or other jaw disorders by performing longitudinal speech analysis.

Sigmatism is a disturbance in the reproduction of consonant sounds from the group of the s-sounds and z-sounds. This condition, as well as some other characteristic pronunciation of syllables in continuous speech, is typical for bruxism. Using longitudinal speech signal analysis, changes related to these symptoms can be detected by comparing with historical data.

The processor may be further configured to analyze an earlier portion of the video data to more accurately diagnose the subject having or predict more precisely the risk of the subject developing, bruxism or other jaw disorders. This may be performed when an analysis of video data was already done and now an earlier portion of the video data is analyzed to perform the diagnosis more accurately.

For example, if a prolonged period of closed jaws is monitored whilst the lips of the user are observed to move, an earlier analysis of the video stream may be used to check if the bones at the back of the jaw have earlier moved relative to each other by monitoring the video signal of the skin at the position of the joint of the jaw at a period (just) after the user closes their mouth. If this is the case, the analysis of bruxism becomes even more specific.

The processor may be configured to remotely actuate the audio channel of the user or remotely actuate the video channel of the user. Thus, the collection of suitable data may be actively managed.

The receiver is for example configured to receive the audio data and video data over a conference call. This provides a user-friendly way to having the analysis.

The invention also provides a computer program which is adapted, when said program is run on a processor, to implement a method comprising: receiving audio data and video data of a subject; identify or analyze one of the audio data and video data indicative of the subject having, or a risk of the subject developing, bruxism or other jaw disorders, and in response thereto, analyze the other of the audio data and video data to diagnose the subject having, or predict the risk of the subject developing, bruxism or other jaw disorders.

The method implemented by the computer program may comprise:
(i) identify or analyze the video data indicative of the subject having, or the risk of developing, bruxism or other jaw disorders by identifying a prolonged period of closed jaws whilst the lips of the user are observed to move; and
   in response, analyzing the audio data to determine if the subject is speaking; or
(ii) analyzing the audio data to diagnose the subject having, or predict the risk of the subject developing, bruxism or other jaw disorders by detecting speech sounds characteristic of speech with a fixed jaw position; and
   in response, analyzing the video data to determine if the subject is speaking with a closed mouth.

The method implemented by the computer program for example comprises: identifying or analyzing the video data indicative of the subject having, or the risk of the subject developing, bruxism or other jaw disorders by identifying a prolonged period of closed jaws whilst the lips of the user are observed to move; and in response, analyzing the audio data to determine if the subject is speaking.

The method implemented by the computer program for example comprises: analyzing the video data to detect yawning as the indication of the subject having, or the risk of the subject developing, bruxism or other jaw disorders; and in response, analyzing the audio data to detect popping or clicking sounds.

The method implemented by the computer program for example comprises: identifying or analyzing the audio data indicative of the subject having, or the risk of the subject developing, bruxism or other jaw disorders by detecting speech sounds characteristic of speech with a fixed jaw position; and in response, analyzing the video data to determine if the subject is speaking with a closed mouth.

After identification or analysis of the video data to detect the subject having, or the risk of the subject developing, bruxism or other jaw disorders, the method may comprise analyzing an earlier portion of the video data to more accurately diagnose the subject having, or predict more precisely the risk of the subject developing, bruxism or other jaw disorders.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a system for detecting bruxism or other jaw disorders;
Fig. 2 shows the method of analysis when the audio analysis is performed first; and
Fig. 3 shows the method of analysis when the video analysis is performed first.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for diagnosing, or predicting a risk of developing, bruxism or other jaw disorders, in which audio data and video data of a subject are analyzed by a processor. One of the audio data and video data is analyzed to detect a potential bruxism or other jaw disorder event, and in response thereto, a particular analysis is performed of the other of the audio data and video data to increase the confidence of a bruxism or other jaw disorder finding. Thus, bruxism or other jaw disorders, or the risk of developing these, are detected or diagnosed based on both video and audio analysis, but data processing is reduced by performing an initial analysis or identification of only video or audio to find candidate data samples for full analysis.

By performing detection based on automated analysis of audio and video, the diagnosis may be made remotely from the subject, over a system for transmitting audio and video, such as a videoconference system. The subject may access such a system simply using a mobile phone, tablet, laptop or indeed a videoconferencing system.

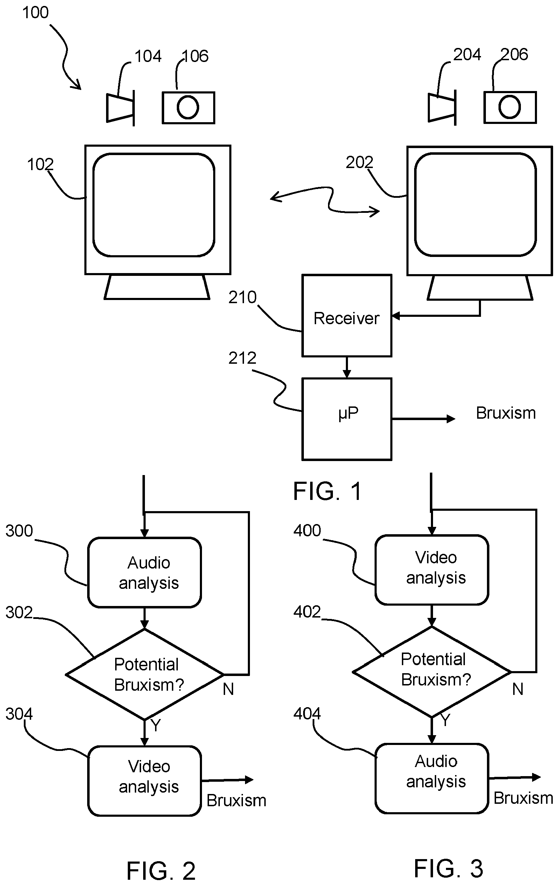

Fig. 1 shows a system 100 for diagnosing or predicting the risk of developing bruxism or other jaw disorders.

In the description below, reference will be made more generally to "bruxism" and for example a "bruxism diagnosis". This should be understood as relating more generally to bruxism or other jaw disorders, unless from the context it is clear than the discussion relates specifically to bruxism. These other jaw disorders may be related to bruxism, for example TMJD may result from bruxism as mentioned above. The term "jaw disorder" is intended to include any abnormal, tooth-tooth contact loading conditions (e.g. jaw clenching, tooth grinding, hence with abnormal contact times or contact pressures). Some jaw disorders may also include abnormal speeds of jaw movement. The jaw disorders to be detected are thus non-physiological in nature.

This particular example makes use of a videoconference system, so that a remote practitioner performs the bruxism diagnosis or the determination of the risk of developing bruxism or other jaw disorders.

There is a user side, comprising a video conferencing system having a display 102 (including audio output), a microphone 104 and a camera 106. Audio and video data streams are thus generated and sent wirelessly to the practitioner side, where the analysis will be conducted.

The practitioner side also comprises a video conferencing system having a display 202 (including audio output), a microphone 204 and a camera 206. The audio and video data streams are received at the practitioner side. Of course, the video conference system allows two-way dialog between the user and the practitioner, so that the practitioner can give instructions to the user.

It is noted however that the system of the invention may be a stand alone system, simply with a camera and microphone for capturing images and sound and directly providing the analysis results to the user. The system may then be fully implemented by a single device, such as a mobile phone running a suitable app.

In the example shown, the practitioner side has a receiver 210 for receiving the audio data and video data of a subject and a processor 212 for processing the audio data and video data to detect bruxism.

The processor identifies one of the audio data and video data indicative of the subject having, or a risk of the subject developing, bruxism or other jaw disorders. Thus, a potential bruxism or jaw disorder event is identified. A potential bruxism or other jaw disorder event may be an event which by itself has indications of a disorder such as bruxism. However, it may instead be an event where a particular further analysis will enable a disorder such as bruxism to be detected reliably. For example, yawning in itself is not indicative of bruxism, but it provides a useful event for further analysis. Thus, the term "potential bruxism or jaw disorder event" may be understood as an event which is indicative of bruxism or other jaw disorder or an event which warrants further investigation because that further investigation is likely to be able to identify bruxism or other disorder if indeed it is present. In the following, "potential bruxism or jaw disorder event" will simply be referred to as a "potential bruxism event".

In response to the detection of a potential bruxism event, a particular analysis of the other of the audio data and video data is selected and performed. This analysis of the other kind of data gives more confidence as to whether or not the potential bruxism event is indeed a bruxism event (and hence more generally a "bruxism or jaw disorder event"). In this analysis it can be determined whether the subject suffers from bruxism or another jaw disorder or the risk of developing it is predicted.

Fig. 2 shows the method of analysis when the audio analysis is performed first as step 300. In step 302 it is determined if there is potential bruxism event and if so, the further video analysis takes place in step 304. Otherwise the video analysis is not performed, and the method returns to the audio analysis.

Fig. 3 shows the method of analysis when the video analysis is performed first as step 400. In step 402 it is determined if there is potential bruxism event and if so, the further audio analysis takes place in step 404. Otherwise the audio analysis is not performed, and the method returns to the video analysis.

Some examples of how to detect bruxism events, or potential bruxism events will be described below. In some cases, the detection is only suitable to look for potential bruxism events (like yawning) and in other cases the detection may be used first (to identify a potential bruxism event) or second (to confirm an already identified potential bruxism event).

### (i) Audio Features Indicative of Bruxism:

The audio signals produced during speech are a combination of the vibrations of the vocal cords, the flow of air, all modulated by the cavity produced by mouth and tongue. For this reason humans are capable of producing many types of audio signals, represented by different audio frequency spectra.

During episodes of daytime bruxism, the subject will talk whilst clenching or grinding the jaws together. Such a limitation on the movement of the jaw will result in a far more limited range of audio spectra, as indeed is familiar from the speech of ventriloquists.

Typical modifications to the audio spectra as a result of bruxism have been analyzed in the literature. For example US 2021/369189 discloses a bruxism detection and correction device which uses audio frequency filters to analyze sounds to determine whether they are associated with a bruxism event.

However, audio analysis alone may not be sufficiently reliable. For example, it may not be certain that a certain audio signal relates to a clenching of the jaws, or simply a result of somebody talking through a closed, but not clenched, jaw.

### (ii) Video Features Indicative of Bruxism:

The video signals recorded during speech are a combination of jaw, lip, teeth and tongue movements and in particular their relative positions. For this reason humans produce many types of video signals to accompany the different audio frequency spectra generated during speech.

During episodes of daytime bruxism, a subject may talk whilst clenching the jaws together. Such a lack of jaw opening can be picked up in a video signal.

However, simply by observing a video, it may again not be certain that a closed jaw relates to a clenching of the jaws, or simply a result of somebody not talking or indeed talking through a closed, but not clenched, jaw.

Another example of visual movement accompanying bruxism is sideways grinding motions of a jaw. These can relatively easily be detected from a video sequence.

Clenching of the jaw can also be detected. During jaw clenching, the bones at the back of the jaw move relative to each other and hence monitoring of the video signal of the skin at the position of the joint of the jaw will be specific of jaw clenching.

There may also be pulsating cheek muscle motion (even sometimes pulsating skin motion on the temples) due to clenching. For detection of such a condition, FFT analysis of the images may be used to extract higher frequency content (>2 Hz) from a video sequence.

Some specific combinations of detection approaches will now be outlined.

A first example, as shown in Fig. 2, is to use the audio stream in order to trigger specific analysis of the video stream. The video stream analysis is only activated after a potential bruxism event is identified in the audio stream.

For example if a prolonged period of audio which is characteristic of speech with a fixed jaw position is detected, the video stream analysis is activated to determine if the user is actually talking with a closed mouth. If this is the case, the chance of the user having daytime bruxism is higher.

Furthermore, if a further analysis of the video stream (before or after the speaking) indicates that the bones at the back of the jaw are moved relative to each other at a period (just) after the user closes their mouth, the analysis of bruxism becomes even more specific. This may be achieved by monitoring the video signal of the skin at the position of the joint of the jaw as explained above.

A second example, as shown in Fig. 3, is to use the video stream in order to trigger a specific analysis of the audio stream. The audio stream analysis is only activated after a potential bruxism event is identified in the video stream. For example if a prolonged period of closed jaws is monitored whilst the lips of the user are observed to move, the audio analysis is performed to check if the user is actually talking. If this is the case, the chance of the user having daytime bruxism is higher. Furthermore, if specific audio spectra are detected which are characteristic of bruxism, the analysis of bruxism becomes even more specific.

Yawing events (beginning, duration, end) can also be recognized from video data to trigger audio-analysis. When a person yawns, the mouth is usually opened to its maximum. Then sounds of a popping or clicking temporomandibular joint (TMJ) can be measured and TMJ disorders (TMJD) detected, which may accompany (and have been caused by) bruxism.

The approach of the invention enables the data volume to be processed to be greatly reduced, as full measurements are only performed when applicable or greatest effect can be expected (e.g. during yawning and sneezing maximum mouth opening occurs which is when sounds relating to TMJD can be measured).

Another interesting option is to use the analysis of the video stream in order to trigger an earlier analysis of the video stream. In order to make efficient use of processing resources, an earlier analysis of the video stream may be made after a potential bruxism event is identified in a video stream.

One example is outlined above, where a prolonged period of closed jaws with moving lips may trigger an earlier analysis of the video stream to check if the bones at the back of the jaw have earlier moved relative to each other. If this is the case, the analysis or prediction of the risk of developing bruxism becomes even more specific.

Research has found that sigmatism (a disturbance in the reproduction of consonant sounds from the group of the s-sounds and z-sounds), as well as certain characteristic pronunciation of syllables in continuous speech, is typical for bruxism. Using longitudinal speech signal analysis, changes such as these can be detected by comparing with historical data.

Additional sensors may be used to make the bruxism determination even more accurate, such as an ear pressure sensor. For example, pressure changes in the external ear canal can be indicative of oral pathologies such as bruxism. A measured external ear pressure can convey information about typical movements involving the jawbones such as jaw up, jaw down, and side-to-side movement of the jaw. This information can then be further used to detect complex movement of oral cavity such as swallowing and yawning, which includes different combinations of typical movement of jawbones. Based on the properties of these movements, a finding of bruxism can be made more accurate.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A system (100) for diagnosing, or predicting a risk of developing, bruxism or other jaw disorders, comprising:
a receiver (210) for receiving audio data and video data of a subject;
a processor (212) configured to:
identify one of the audio data and video data indicative of the subject having, or a risk of the subject developing, bruxism or other jaw disorders, and in response thereto,
analyze the other of the audio data and video data to diagnose the subject having, or predict the risk of the subject developing, bruxism or other jaw disorders.

2. The system of claim 1, wherein the processor is configured to:
identify the video data indicative of the subject having, or the risk of the subject developing, bruxism or other jaw disorders by identifying a prolonged period of closed jaws whilst the lips of the user are observed to move; and
in response, analyze the audio data to determine if the subject is speaking.

3. The system of claim 2, wherein the processor is configured to:
analyze the spectra of the audio data to detect features which are indicative of the subject having, or the risk of the subject developing, bruxism or other jaw disorders.

4. The system of any one of claims 1 to 3, wherein the processor is configured to
analyze the video data to detect yawning as the indication of the subject having, or the risk of the subject developing, bruxism or other jaw disorders; and
in response, analyze the audio data to detect popping or clicking sounds.

5. The system of claim 1, wherein the processor is configured to:
identify the audio data indicative of the subject having, or the risk of the subject developing, bruxism or other jaw disorders by detecting speech sounds characteristic of speech with a fixed jaw position; and
in response, analyze the video data to determine if the subject is speaking with a closed mouth.

6. The system of any one of claims 1 to 5, wherein the processor is configured to analyze the video data to diagnose the subject having, or predict the risk of the subject developing, bruxism or other jaw disorders by identifying recurring sideways grinding motions of a jaw.

7. The system of any one of claims 1 to 6, wherein the processor is configured to analyze the video data to diagnose the subject having, or predict the risk of the subject developing, bruxism or other jaw disorders by monitoring facial skin and/or muscle motion at the position of the joint of the jaw and/or movement of the ears to detect jaw clenching.

8. The system of any one of claims 1 to 7, wherein the processor is configured to analyze the audio data to diagnose the subject having, or predict the risk of the subject developing, bruxism or other jaw disorders by performing longitudinal speech analysis.

9. The system of any one of claims 1 to 8, wherein the processor is further configured to analyze an earlier portion of the video data to more accurately diagnose the subject having, or predict more precisely the risk of the subject developing, bruxism or other jaw disorders.

10. The system of any one of claims 1 to 9, wherein the processor is configured to remotely actuate the audio channel of the user or remotely actuate the video channel of the user.

11. The system of any one of claims 1 to 10, wherein the receiver is configured to receive the audio data and video data over a conference call.

12. A computer program which is adapted, when said program is run on a processor, to implement a method for diagnosing or, predicting a risk of developing, bruxism or other jaw disorders, comprising:
receiving audio data and video data of a subject;
identify one of the audio data and video data indicative of the subject having, or a risk of the subject developing, bruxism or other jaw disorders, and in response thereto,
analyze the other of the audio data and video data to diagnose the subject having, or predict the risk of the subject developing, bruxism or other jaw disorders.

13. The computer program of claim 12, wherein the method implemented by the computer program comprises:
(i) identify the video data indicative of the subject having, or the risk of developing, bruxism or other jaw disorders by identifying a prolonged period of closed jaws whilst the lips of the user are observed to move; and
in response, analyzing the audio data to determine if the subject is speaking; or
(ii) analyzing the audio data to diagnose the subject having, or predict the risk of the subject developing, bruxism or other jaw disorders by detecting speech sounds characteristic of speech with a fixed jaw position; and
in response, analyzing the video data to determine if the subject is speaking with a closed mouth.

14. The computer program of claim 12 or 13, wherein the method implemented by the computer program comprises:
analyzing the video data to detect yawning as the indication of the subject having, or the risk of the subject developing, bruxism or other jaw disorders; and
in response, analyzing the audio data to detect popping or clicking sounds.

15. The computer program of any one of claims 12 to 14, wherein the method implemented by the computer program comprises analyzing an earlier portion of the video data to more accurately diagnose the subject having, or predict more precisely the risk of the subject developing, bruxism or other jaw disorders.
